# EUROPEAN PATENT SPECIFICATION

(11) **EP 2 750 687 B1**
(45) Date of publication and mention of the grant of the patent: **06.12.2017**
(21) Application number: 12828087.2
(22) Date of filing: 31.08.2012
(51) Int. Cl.: A61K 38/10, A61P 11/00

(54) **AMPHIPHILIC PEPTIDES FOR THORACIC AIR LEAKAGE OCCLUSION**
AMPHIPHILE PEPTIDE FÜR THORAX-LUFTLECKOKKLUSION
PEPTIDES AMPHIPHILES POUR L'OCCLUSION DE FUITE D'AIR THORACIQUE

(30) Priority: 02.09.2011 US 201161530695 P
(43) Date of publication of application: 09.07.2014
(73) Proprietor: 3-D Matrix, Ltd., Chiyoda-ku Tokyo 102-0083 (JP)
(72) Inventor: KOBAYASHI, Satoru, Chigasaki City Kanagawa 253-0085 (JP); OKADA, Satoshi, Suita City Osaka 565-0878 (JP); TAKAMURA, Kentaro, Tokoyo 152-0011 (JP)
(74) Representative: HGF Limited
(86) International application number: PCT/IB2012/002172
(87) International publication number: WO 2013/030673

(56) References cited:
- WO-A1-2006/116524
- WO-A1-2009/072556
- WO-A1-2010/041636
- WO-A2-2008/073392
- US-A1- 2011 002 880
- US-A1- 2011 201 541
- HIRAI, K. ET AL.: 'The fundamental study of Matrigel (PuraMatrix TM) for the hemostasis of bleeding from pulmonary artery and vein or the prevention of lung fistel' GEN THORAC CARDIOVASC SURG vol. 59, 10 September 2011, page 600, XP008173637

### SEQUENCE LISTING

<110> 3-D Matrix Ltd.
<120> AMPHIPHILIC PEPTIDES FOR THORACIC AIR LEAKAGE OCCLUSION
<130> TDX-003.25
<150> US 61/530,695
   <151> 2011-09-02
<160> 9
<170> PatentIn version 3.5
<210> 1
   <211> 16
   <212> PRT
   <213> Artificial sequence
<220>
   <223> Synthetic peptide
<220>
   <221> MOD_RES
   <222> (1)..(1)
   <223> ACETYLATION
<220>
   <221> MOD_RES
   <222> (16)..(16)
   <223> AMIDATION
<400> 1
   Arg Ala Asp Ala Arg Ala Asp Ala Arg Ala Asp Ala Arg Ala Asp Ala
   1               5                   10                  15
<210> 2
   <211> 13
   <212> PRT
   <213> Artificial sequence
<220>
   <223> Synthetic peptide
<220>
   <221> MOD_RES
   <222> (1)..(1)
   <223> ACETYLATION
<220>
   <221> MOD_RES
   <222> (13)..(13)
   <223> AMIDATION
<400> 2
   Ile Glu Ile Lys Ile Glu Ile Lys Ile Glu Ile Lys Ile
   1               5                   10
<210> 3
   <211> 12
   <212> PRT
   <213> Artificial sequence
<220>
   <223> Synthetic peptide
<220>
   <221> MOD_RES
   <222> (1)..(1)
   <223> ACETYLATION
<220>
   <221> MOD_RES
   <222> (12)..(12)
   <223> AMIDATION
<400> 3
   Lys Leu Asp Leu Lys Leu Asp Leu Lys Leu Asp Leu
   1               5                   10
<210> 4
   <211> 16
   <212> PRT
   <213> Artificial sequence
<220>
   <223> Synthetic peptide
<400> 4
   Arg Ala Asp Arg Ala Asp Arg Ala Asp Arg Ala Asp Arg Ala Asp Arg
   1               5                   10                  15
<210> 5
   <211> 16
   <212> PRT
   <213> Artificial sequence
<220>
   <223> Synthetic peptide
<400> 5
   Ala Asp Arg Ala Asp Arg Ala Asp Arg Ala Asp Arg Ala Asp Arg Ala
   1               5                   10                  15
<210> 6
   <211> 16
   <212> PRT
   <213> Artificial sequence
<220>
   <223> Synthetic peptide
<400> 6
   Asp Arg Ala Asp Arg Ala Asp Arg Ala Asp Arg Ala Asp Arg Ala Asp
   1 5 10 15
<210> 7
   <211> 200
   <212> PRT
   <213> Artificial sequence
<220>
   <223> Synthetic peptide
<220>
   <221> MOD_RES
   <222> (1)..(1)
   <223> ACETYLATION
<220>
   <221> MISC_FEATURE
   <222> (5)..(196)
   <223> Any one or more RADA repeats may be absent.
<220>
   <221> MOD_RES
   <222> (200)..(200)
   <223> AMIDATION
<400> 7
   Arg Ala Asp Ala Arg Ala Asp Ala Arg Ala Asp Ala Arg Ala Asp Ala
   1 5 10 15
   Arg Ala Asp Ala Arg Ala Asp Ala Arg Ala Asp Ala Arg Ala Asp Ala
   20 25 30
   Arg Ala Asp Ala Arg Ala Asp Ala Arg Ala Asp Ala Arg Ala Asp Ala
   35 40 45
   Arg Ala Asp Ala Arg Ala Asp Ala Arg Ala Asp Ala Arg Ala Asp Ala
   50 55 60
   Arg Ala Asp Ala Arg Ala Asp Ala Arg Ala Asp Ala Arg Ala Asp Ala
   65 70 75 80
   Arg Ala Asp Ala Arg Ala Asp Ala Arg Ala Asp Ala Arg Ala Asp Ala
   85 90 95
   Arg Ala Asp Ala Arg Ala Asp Ala Arg Ala Asp Ala Arg Ala Asp Ala
   100 105 110
   Arg Ala Asp Ala Arg Ala Asp Ala Arg Ala Asp Ala Arg Ala Asp Ala
   115 120 125
   Arg Ala Asp Ala Arg Ala Asp Ala Arg Ala Asp Ala Arg Ala Asp Ala
   130 135 140
   Arg Ala Asp Ala Arg Ala Asp Ala Arg Ala Asp Ala Arg Ala Asp Ala
   145 150 155 160
   Arg Ala Asp Ala Arg Ala Asp Ala Arg Ala Asp Ala Arg Ala Asp Ala
   165 170 175
   Arg Ala Asp Ala Arg Ala Asp Ala Arg Ala Asp Ala Arg Ala Asp Ala
   180 185 190
   Arg Ala Asp Ala Arg Ala Asp Ala
   195 200
<210> 8
   <211> 200
   <212> PRT
   <213> Artificial sequence
<220>
   <223> Synthetic peptide
<220>
   <221> MOD_RES
   <222> (1)..(1)
   <223> ACETYLATION
<220>
   <221> MISC_FEATURE
   <222> (5)..(196)
   <223> Any one or more IEIK repeats may be absent.
<220>
   <221> MOD_RES
   <222> (200)..(200)
   <223> AMIDATION
<400> 8
   Ile Glu Ile Lys Ile Glu Ile Lys Ile Glu Ile Lys Ile Glu Ile Lys
   1 5 10 15
   Ile Glu Ile Lys Ile Glu Ile Lys Ile Glu Ile Lys Ile Glu Ile Lys
   20 25 30
   Ile Glu Ile Lys Ile Glu Ile Lys Ile Glu Ile Lys Ile Glu Ile Lys
   35 40 45
   Ile Glu Ile Lys Ile Glu Ile Lys Ile Glu Ile Lys Ile Glu Ile Lys
   50 55 60
   Ile Glu Ile Lys Ile Glu Ile Lys Ile Glu Ile Lys Ile Glu Ile Lys
   65                  70                  75                  80
   Ile Glu Ile Lys Ile Glu Ile Lys Ile Glu Ile Lys Ile Glu Ile Lys
                   85                  90                  95
   Ile Glu Ile Lys Ile Glu Ile Lys Ile Glu Ile Lys Ile Glu Ile Lys
               100                 105                 110
   Ile Glu Ile Lys Ile Glu Ile Lys Ile Glu Ile Lys Ile Glu Ile Lys
           115                 120                 125
   Ile Glu Ile Lys Ile Glu Ile Lys Ile Glu Ile Lys Ile Glu Ile Lys
       130                 135                 140
   Ile Glu Ile Lys Ile Glu Ile Lys Ile Glu Ile Lys Ile Glu Ile Lys
   145                 150                 155                 160
   Ile Glu Ile Lys Ile Glu Ile Lys Ile Glu Ile Lys Ile Glu Ile Lys
                   165                 170                 175
   Ile Glu Ile Lys Ile Glu Ile Lys Ile Glu Ile Lys Ile Glu Ile Lys
               180                 185                 190
   Ile Glu Ile Lys Ile Glu Ile Lys
           195                 200
<210> 9
   <211> 200
   <212> PRT
   <213> Artificial sequence
<220>
   <223> Synthetic peptide
<220>
   <221> MOD_RES
   <222> (1)..(1)
   <223> ACETYLATION
<220>
   <221> MISC_FEATURE
   <222> (5)..(196)
   <223> Any one or more KLDL repeats may be absent.
<220>
   <221> MOD_RES
   <222> (200)..(200)
   <223> AMIDATION
<400> 9
   Lys Leu Asp Leu Lys Leu Asp Leu Lys Leu Asp Leu Lys Leu Asp Leu
   1               5                   10                  15
   Lys Leu Asp Leu Lys Leu Asp Leu Lys Leu Asp Leu Lys Leu Asp Leu
               20                  25                  30
   Lys Leu Asp Leu Lys Leu Asp Leu Lys Leu Asp Leu Lys Leu Asp Leu
           35                  40                  45
   Lys Leu Asp Leu Lys Leu Asp Leu Lys Leu Asp Leu Lys Leu Asp Leu
       50                  55                  60
   Lys Leu Asp Leu Lys Leu Asp Leu Lys Leu Asp Leu Lys Leu Asp Leu
   65                  70                  75                  80
   Lys Leu Asp Leu Lys Leu Asp Leu Lys Leu Asp Leu Lys Leu Asp Leu
                   85                  90                  95
   Lys Leu Asp Leu Lys Leu Asp Leu Lys Leu Asp Leu Lys Leu Asp Leu
               100                 105                 110
   Lys Leu Asp Leu Lys Leu Asp Leu Lys Leu Asp Leu Lys Leu Asp Leu
           115                 120                 125
   Lys Leu Asp Leu Lys Leu Asp Leu Lys Leu Asp Leu Lys Leu Asp Leu
       130                 135                 140
   Lys Leu Asp Leu Lys Leu Asp Leu Lys Leu Asp Leu Lys Leu Asp Leu
   145                 150                 155                 160
   Lys Leu Asp Leu Lys Leu Asp Leu Lys Leu Asp Leu Lys Leu Asp Leu
                   165                 170                 175
   Lys Leu Asp Leu Lys Leu Asp Leu Lys Leu Asp Leu Lys Leu Asp Leu
               180                 185                 190
   Lys Leu Asp Leu Lys Leu Asp Leu
           195                 200

## Description

### TECHNICAL FIELD

The present invention relates to a pulmonary air leakage occluding agent comprising a self-assembling peptide hydrogel.

### BACKGROUND OF THE INVENTION

Pulmonary air leaks due to thoracic trauma, thoracic and pulmonary surgery, lung cancer, and pyothorax remain challenging clinical problems. Lung surgeries include open surgery, thoracoscopic surgery, and bronchoscopic surgery. Lung surgeries also include lung transplants. During and following lung surgery, air often leaks for sutured sites, resected surfaces of lungs, bronchial anastomosis sites, and sites of bronchorrhaphy (suture of a wound of a bronchus). Such air leaks cause collapse of the lungs (pneumothorax) and empyema.

Traditionally, pulmonary air leaks have been treated with the insertion, through the chest wall, of chest tubes through which vacuum is applied to maintain lung volume until the air leak has sealed. More recently, products have been developed and used in the treatment of pulmonary air leaks. These products include oxidized cellulose, polyglycolic acid, and fibrin glues. Such products are typically applied directly to the site or sites of air leakage.

Existing products for the treatment of pulmonary air leaks have certain disadvantages. For example, fibrin glue, consisting of a biological substance, presents a risk of infection. Moreover, fibrin glue frequently solidifies during its application, thereby limiting its efficacy and ease of use. Both oxidized cellulose and polyglycolic acid have been found to have only limited efficacy.

US 2011/0002880 and US 2011/0201541 disclose certain self-assembling peptides useful for wound healing, skin reconstruction, and tissue occlusion to prevent leakage of body fluids (e.g., to achieve hemostasis).

### SUMMARY OF THE INVENTION

The present invention is defined by the claims. The present inventors have completed this invention upon finding that a pulmonary air leakage occluding effect equivalent to or greater than that of existing pulmonary air leakage occluding agents is exhibited when a self-assembling peptide hydrogel utilized as a scaffold for cell culture is applied for pulmonary air leakage occlusion.

Specifically, the disclosure comprising the invention relates to a pulmonary air leakage occluding agent containing a peptide, wherein the peptide is an amphiphilic peptide having 8-200 amino acid residues with the hydrophilic amino acids and hydrophobic amino acids alternately bonded, and is a self-assembling peptide exhibiting a beta-sheet structure in aqueous solution in the presence of physiological pH and/or in the presence of a cation.

In an embodiment, the peptide is 16 amino acid residues long. In one instance, the peptide comprises a repeated sequence arginine-alanine-aspartic acid (RAD). In one instance, the peptide consists essentially of a repeated sequence arginine-alanine-aspartic acid (RAD). In one instance, the peptide is a repeated sequence arginine-alanine-aspartic acid (RAD).

In one instance, the peptide comprises a repeated sequence arginine-alanine-aspartic acid-alanine (RADA). In one instance, the peptide consists essentially of a repeated sequence arginine-alanine-aspartic acid-alanine (RADA). In one instance, the peptide is a repeated sequence arginine-alanine-aspartic acid-alanine (RADA).

In one instance, the peptide has the amino acid sequence Ac-(RADA)₄-CONH₂ (SEQ ID NO:1), Ac-(IEIK)₃I-CONH₂ (SEQ ID NO:2), or Ac-(KLDL)₃-CONH₂ (SEQ ID NO:3).

In one instance, the peptide has the amino acid sequence (RAD)₅R (SEQ ID NO:4), (ADR)₅A (SEQ ID NO:5), or (DRA)₅D (SEQ ID NO:6).

In one instance, the peptide is provided as an aqueous solution of 0.5 % to 2.5 % (weight of peptide to volume).

An aspect is the above peptide for use in a method of occluding a pulmonary air leak. The method includes the step of applying to a site of pulmonary air leak an effective amount of an amphiphilic peptide having 8-200 amino acid residues with the hydrophilic amino acids and hydrophobic amino acids alternately bonded, and is a self-assembling peptide exhibiting a beta-sheet structure in aqueous solution in the presence of physiological pH and/or in the presence of a cation.

In an embodiment, the peptide is 16 amino acid residues long.

In one instance, the peptide comprises a repeated sequence arginine-alanine-aspartic acid (RAD). In one instance, the peptide consists essentially of a repeated sequence arginine-alanine-aspartic acid (RAD). In one instance, the peptide is a repeated sequence arginine-alanine-aspartic acid (RAD).

In one instance, the peptide comprises a repeated sequence arginine-alanine-aspartic acid-alanine (RADA). In one instance, the peptide consists essentially of a repeated sequence arginine-alanine-aspartic acid-alanine (RADA). In one instance, the peptide is a repeated sequence arginine-alanine-aspartic acid-alanine (RADA).

In one instance, the peptide has the amino acid sequence Ac-(RADA)₄-CONH₂ (SEQ ID NO:1), Ac-(IEIK)₃I-CONH₂ (SEQ ID NO:2), or Ac-(KLDL)₃-CONH₂ (SEQ ID NO:3).

In one instance, the peptide has the amino acid sequence (RAD)₅R (SEQ ID NO:4), (ADR)₅A (SEQ ID NO:5), or (DRA)₅D (SEQ ID NO:6).

In one instance, the peptide is provided as an aqueous solution of about 0.5 % to about 3 % (weight of peptide to volume).

In one embodiment, the peptide is applied to lungs.

In one embodiment, the peptide is applied to a bronchus.

In one embodiment, the peptide is applied thoracoscopically.

In one embodiment, the peptide is applied bronchoscopically.

In certain embodiments the pulmonary air leak occluding agent also includes at least one small molecule drug useful to treat a condition selected from cancer, inflammation, and infection.

### BRIEF DESCRIPTION OF THE FIGURES

Figure 1 is a group of three chest X-rays of a mini-pig obtained before (left), immediately after (middle), and 10 days after treatment of a surgically created pulmonary air leak with a self-assembling peptide hydrogel of the invention.
Figure 2 is a pair of photomicrographs depicting histopathologic appearance of lung tissue with a surgically created puncture and occlusion by self-assembling peptide hydrogel. The image on the right is a detail of the circled portion of the image on the left. Self-assembling peptide hydrogel is indicated by a circle in the image on the right.

### DETAILED DESCRIPTION OF THE INVENTION

Self-assembling peptides have a property whereby the peptide molecules form regularly arranged self-assemblies according to their amino acid sequence. In recent years, these have attracted much attention as novel materials because of their physical, chemical, and biological properties.

Self-assembling peptides of the invention have an alternating structure of electrically charged hydrophilic amino acids and electrically neutral hydrophobic amino acids, and alternating distribution of positive charge and negative charge, whereby they adopt a beta-sheet structure at physiological pH and salt concentration.

Hydrophilic amino acids that can be used include acidic amino acids such as aspartic acid and glutamic acid, and basic amino acids such as arginine, lysine, histidine, and ornithine.

As hydrophobic amino acids there may be used alanine, valine, leucine, isoleucine, methionine, phenylalanine, tyrosine, tryptophan, serine, threonine, or glycine.

The self-assembly of such peptides occurs under the following conditions.
(1) The peptide molecules adopt a beta-sheet structure in aqueous solution, wherein the charged hydrophilic amino acids and electrically neutral hydrophobic amino acids are maldistributed on the two sides of the peptide molecules.
(2) The beta-sheet structure results in a complementary electrical distribution between adjacent molecules.
(3) The beta-sheet structure leads to sufficient hydrophobic bonding between adjacent molecules.
(4) The electrical charge of the amino acid side chains is screened by monovalent inorganic salts.
(5) The molecules are electrostatically neutral near the isoelectric point of the peptide.

It is believed that self-assembly occurs by the following mechanism when these conditions are all satisfied.
(1) The alternating distribution of positive charge and negative charge in the peptide molecules causes attraction between the molecules.
(2) Hydrophobic bonds are formed between the neutral amino acid side chains of adjacent molecules.
(3) The positive/negative electrical distribution results in complementary alignment between adjacent molecules, and associative force between the molecules is strengthened.
(4) The molecular aggregates gradually extend, forming nanofibers.

The nanofibers are superfine fibers with thicknesses of about 10 nm to 20 nm, and they aggregate to form meshwork and exhibit a macroscopically gel-like form.

The gel network structure strongly resembles a natural extracellular matrix (ECM) in terms of its fiber size and pore size, and its use as a scaffold for cell culture is being studied.

Since the peptide hydrogel is biodegradable and its decomposition product does not adversely affect tissue, while it is also highly bioabsorbable, it is suitable for cellular engraftment and growth.

Because self-assembling peptides are chemical synthetic products, rather than products isolated from biological sources, they do not carry the risk of infectious disease from animal-derived products, including animal viruses and other infectious agents such as the agent of mad cow disease (bovine spongiform encephalopathy, BSE).

In this pulmonary air leakage occluding agent, the peptide is preferably a self-assembling peptide having a repeating sequence arginine-alanine-aspartic acid-alanine (RADA); a repeating sequence isoleucine-glutamic acid-isoleucine-lysine (IEIK); or a repeating sequence lysine-leucine-aspartic acid-leucine (KLDL). In an embodiment, it is a self-assembling peptide comprising the amino acid sequence Ac-(RADA)₄-CONH₂ (SEQ ID NO:1). In one instance, it is a self-assembling peptide comprising the amino acid sequence Ac-(IEIK)₃I-CONH₂ (SEQ ID NO:2). In one instance, it is a self-assembling peptide comprising the amino acid sequence Ac-(KLDL)₃-CONH₂ (SEQ ID NO:3).

The pulmonary air leakage occluding agent of the invention will now be explained in detail.

The main component of the pulmonary air leakage occluding agent of the disclosure comprising the invention is a self-assembling peptide which is an amphiphilic peptide having 8-200 amino acid residues with the hydrophilic amino acids and hydrophobic amino acids alternately bonded, and it exhibits a beta-sheet structure in aqueous solution in the presence of physiological pH and/or a cation.

According to the invention, physiological pH is pH 6-8, preferably pH 6.5-7.5 and more preferably pH 7.3-7.5.

A "cation" as used herein is a positively charged ion, for example, sodium ion (Na⁺) or potassium ion (K⁺). In one instance, the cation is present at a concentration of about 5 mM to 5 M. A cation can be a single cation or any combination of cations.

Self-assembling peptides used for the disclosure can be represented by the following four general formulas.

((XY)ₗ-(ZY)ₘ)ₙ (I)

((YX)ₗ-(YZ)ₘ)ₙ (II)

((ZY)ₗ-(XY)ₘ)ₙ (III)

((YZ)ₗ-(YX)ₘ)ₙ (IV)

In formulas (I)-(IV), X represents an acidic amino acid, Y represents a hydrophobic amino acid, Z represents a basic amino acid, and l, m, and n are all integers, wherein n × (l + m) < 200.

Of course, it is not required that a peptide of the invention begin and end with complete repeating unit. That is, only a portion of any given repeating unit may be present at either one or both ends of a peptide of the invention. For example, a peptide made up primarily of RADA repeating units may begin with N-terminal A, DA, or ADA; likewise, a peptide made up primarily of RADA repeating units may end with C-terminal R, RA, or RAD.

The N-terminals may be acetylated, and the C-terminals may be amidated.

Hydrophilic amino acids that can be used include acidic amino acids such as aspartic acid and glutamic acid, and basic amino acids such as arginine, lysine, histidine and ornithine. As hydrophobic amino acids there may be used alanine, valine, leucine, isoleucine, methionine, phenylalanine, tyrosine, tryptophan, serine, threonine or glycine.

Preferred among these self-assembling peptides are self-assembling peptides having the repeating sequence arginine-alanine-aspartic acid-alanine (RADA), and such peptide sequences are represented by Ac-(RADA)ₚ-CONH₂ (p=2-50) (SEQ ID NO:7). There are also preferred self-assembling peptides having the repeating sequence isoleucine-glutamine acid-isoleucine-lysine (IEIK), and such peptide sequences are represented by Ac-(IEIK)ₚI-CONH₂ (p= 2-50) (SEQ ID NO:8). There are additionally preferred self-assembling peptides having the repeating sequence lysine-leucine-aspartic acid-leucine (KLDL), and such peptide sequences are represented by Ac-(KLDL)ₚ-CONH₂ (p =2-50) (SEQ ID NO:9). These self-assembling peptides may be composed of 8-20 amino acid residues, with 8-32 residue self-assembling peptides being preferred, and self-assembling peptides having 12-16 residues being more preferred. In an embodiment, the peptide is 16 amino acid residues long.

As specific examples of self-assembling peptides according to the disclosure there may be mentioned peptide RAD16-I having the sequence Ac-(RADA)₄-CONH₂ (SEQ ID NO:1), peptide IEIK13 having the sequence Ax-(IEIK)₃I-CONH₂ (SEQ ID NO:2), and peptide KLD having the sequence Ac-(KLDL)₃-CONH₂ (SEQ ID NO:3). A 1 % aqueous solution of RAD6-I is available as the product PuraMatrix™ by 3D-Matrix Co., Ltd. PuraMatrix™ contains 1 % peptide having the sequence Ac-(RADA)₄-CONH₂ (SEQ ID NO:1), with hydrogen ion and chloride ion.

In one instance, the peptide has the amino acid sequence (RAD)₅R (SEQ ID NO:4), (ADR)₅A (SEQ ID NO:5), or (DRA)₅D (SEQ ID NO:6). The N-terminals may be acetylated, and the C-terminals may be amidated, similar to SEQ ID NOs:1-3.

Peptides in accordance with the invention can be prepared using standard peptide synthetic methods and apparatus, e.g., using a programmable automated peptide synthesizer. Peptide synthesizers and reagents for use with same are readily available from any of a number of commercial suppliers, e.g., Applied Biosystems.

PuraMatrix™, IEIK13, and KLD are oligopeptides of 12-16 amino acid residues and having a length of about 5 nm. Although their solutions are liquid at acidic pH, at a concentration of at least about 0.1 % (w/v) the peptides undergo self-organization upon change to neutral pH, forming nanofibers with diameters of about 10 nm, causing gelling of the peptide solutions.

PuraMatrix™ is an amphiphilic peptide having an amino acid sequence with alternate repeats of positively charged arginine and negatively charged aspartic acid as hydrophilic amino acids, and alanine as a hydrophobic amino acid. IEIK13 is an amphiphilic peptide having an amino acid sequence with alternate repeats of positively charged lysine and negatively charged glutamic acid as hydrophilic amino acids and isoleucine as a hydrophobic amino acid. KLD is an amphiphilic peptide having an amino acid sequence with alternate repeats of positively charged lysine and negatively charged aspartic acid as hydrophilic amino acids and leucine as a hydrophobic amino acid. The self-assembly of these peptides is due to hydrogen bonding and hydrophobic bonding between the peptide molecules by the amino acids composing the peptides.

In the self-assembling peptides used for the invention, the nanofiber diameter is 10-20 nm and the pore size is 5-200 nm, as averages. These numerical value ranges are approximately the same as collagen, which is a natural extracellular matrix.

Physiological pH and salt concentration are conditions for self-assembly of the self-assembling peptides of the invention. The presence of a monovalent alkali metal ion promotes gelling. Once gelling has occurred, the gel does not decompose, even under common protein-denaturing conditions such as exposure to high temperature or denaturing agents such as acids, alkalis, proteases, urea, guanidine hydrochloride or the like.

These self-assembling peptides, such as PuraMatrix™, are peptide sequences lacking a distinct physiologically active motif, and therefore intrinsic cell function is not impaired. Physiologically active motifs control numerous intracellular phenomena such as transcription, and the presence of physiologically active motifs can lead to phosphorylation of intracytoplasmic or cell surface proteins by enzymes that recognize the motifs. When a physiologically active motif is present in a peptide agent, transcription of proteins with various functions can be activated or suppressed. The self-assembling peptides, such as PuraMatrix™, lack such physiologically active motifs and therefore do not carry this risk.

Furthermore, a self-assembling peptide composed of natural amino acids also has satisfactory biocompatibility and biodegradability, and it has been reported that infusion of PuraMatrix™ into murine cardiac muscle, for example, results in infiltration of cells into the PuraMatrix™ and formation of normal tissue. The decomposition time differs depending on the conditions such as the location of infusion, but the fibers decompose and are excreted by about 2 to 8 weeks after infusion.

The pulmonary air leakage occluding agent of the invention may further contain one or more small molecule drugs. As used herein, a small molecule drug is an organic molecule of up to 1 kDa in molecular weight having pharmaceutical activity.

There are no particular restrictions on such small molecule drugs, and these may include, without limitation, glucose, saccharose, purified saccharose, lactose, maltose, trehalose, dextran, iodine, lysozyme chloride, dimethylisopropylazulene, tretinoin tocoferil, povidone iodine, alprostadil alfadex, anise alcohol, isoamyl salicylate, alpha, alpha-dimethylphenylethyl alcohol, bacdanol, helional, sulfazin silver, bucladesine sodium, alprostadil alfadex, gentamycin sulfate, tetracycline hydrochloride, sodium fusidate, mupirocin calcium hydrate and isoamyl benzoate.

The small molecule drug can be an anti-cancer agent. As used herein, an anti-cancer agent refers to a chemotherapeutic agent or other small molecule or radionuclide useful for killing cancer cells. Examples of chemotherapeutic agents include 13-cis-Retinoic Acid, 2-Chlorodeoxyadenosine, 5-Azacitidine, 5-Fluorouracil, (5-FU), 6-Mercaptopurine, (6-MP), 6-Thioguanine (6-TG), Abraxane, Accutane®, Actinomycin-D, Adriamycin®, Adrucil®, Afinitor®, Agrylin®, Ala-Cort®, Aldesleukin, ALIMTA, Alitretinoin, Alkaban-AQ®, Alkeran®, All-Transretinoic Acid, Altretamine, Amethopterin, Amifostine, Aminoglutethimide, Anagrelide, Anandron®, Anastrozole, Arabinosylcytosine, Ara C, Aranesp®, Aredia®, Arimidex®, Aromasin®, Arranon®, Arsenic Trioxide, Arzerra™, Asparaginase, ATRA, Avastin®, Azacitidine, BCG, BCNU, Bendamustine, Bexarotene, BEXXAR®, Bicalutamide, BiCNU, Blenoxane®, Bleomycin, Busulfan, Busulfex®, C225, Calcium Leucovorin, Camptosar®, Camptothecin-11, Capecitabine, Carac™, Carboplatin, Carmustine, Carmustine Wafer, Casodex®, CC-5013, CC1-779, CCNU, CDDP, CeeNU, Cerubidine®, Chlorambucil, Cisplatin, Citrovorum Factor, Cladribine, Cortisone, Cosmegen®, CPT-11, Cyclophosphamide, Cytadren®, Cytarabine, Cytarabine Liposomal, Cytosar-U®, Cytoxan®, Dacarbazine, Dacogen, Dactinomycin, Darbepoetin Alfa, Dasatinib, Daunomycin, Daunorubicin, Daunorubicin Hydrochloride, Daunorubicin Liposomal, DaunoXome®, Decadron, Decitabine, Delta-Cortef®, Deltasone®, Denileukin, Diftitox, DepoCyt™, Dexamethasone, Dexamethasone Acetate, Dexamethasone Sodium Phosphate, Dexasone, Dexrazoxane, DHAD, DIC, Diodex, Docetaxel, Doxil®, Doxorubicin, Doxorubicin Liposomal, Droxia™, DTIC, DTIC-Dome®, Duralone®, Efudex®, Eligard™, Ellence™, Eloxatin™, Elspar®, Emcyt®, Epirubicin, Erbitux, Erlotinib, Erwinia, L-asparaginase, Estramustine, Ethyol, Etopophos®, Etoposide, Etoposide Phosphate, Eulexin®, Everolimus, Evista®, Exemestane, Fareston®, Faslodex®, Femara®, Filgrastim, Floxuridine, Fludara®, Fludarabine, Fluoroplex®, Fluorouracil, Fluoxymesterone, Flutamide, Folinic Acid, FUDR®, Fulvestrant, Gefitinib, Gemcitabine, Gemzar, Gleevec™, Gliadel® Wafer, Goserelin, Halotestin®, Herceptin®, Hexadrol, Hexalen®, Hexamethylmelamine (HMM), Hycamtin®, Hydrea®, Hydrocort Acetate®, Hydrocortisone, Hydrocortisone Sodium Phosphate, Hydrocortisone Sodium Succinate, Hydrocortone Phosphate, Hydroxyurea, Tiuxetan, Idamycin®, Idarubicin, Ifex®, Ifosfamide, Imatinib mesylate, Imidazole Carboxamide, Intron A®, Iressa®, Irinotecan, Isotretinoin, Ixabepilone, Ixempra™, Kidrolase (t), Lanacort®,L-asparaginase, LCR, Lenalidomide, Letrozole, Leucovorin, Leukeran, Leukine™, Leuprolide, Leurocristine, Leustatin™, Liposomal Ara-C, Liquid Pred®, Lomustine, L-PAM, L-Sarcolysin, Lupron®, Lupron Depot®, Matulane®, Maxidex, Mechlorethamine, Mechlorethamine Hydrochloride, Medralone®, Medrol®, Megace®, Megestrol, Megestrol Acetate, Melphalan, Mercaptopurine, Mesna, Mesnex™, Methotrexate, Methotrexate Sodium, Methylprednisolone, Meticorten®, Mitomycin, Mitomycin-C, Mitoxantrone, M-Prednisol®, MTC, MTX, Mustargen®, Mustine, Mutamycin®, Myleran®, Mylocel™, Mylotarg®, Navelbine®, Nelarabine, Neosar®, Neulasta™, Neumega®, Neupogen®, Nexavar®, Nilandron®, Nilotinib, Nilutamide, Nipent®, Nitrogen Mustard, Novaldex®, Novantrone®, Nplate, Octreotide, Octreotide acetate,Oncospar®, Oncovin®, Ontak®, Onxal™, Oprelvekin, Orapred®, Orasone®, Oxaliplatin, Paclitaxel, Pamidronate, Panretin®, Paraplatin®, Pazopanib, Pediapred®, Pegaspargase, Pegfilgrastim, PEG-L-asparaginase, PEMETREXED, Pentostatin, Phenylalanine Mustard, Platinol®, Platinol-AQ®, Prednisolone, Prednisone, Prelone®, Procarbazine, Prolifeprospan 20 with Carmustine Implant, Purinethol®, Raloxifene, Revlimid®, Rheumatrex®, Romiplostim, Rubex®, Rubidomycin hydrochloride, Sandostatin®, Sandostatin LAR®, Sargramostim, Solu-Cortef®, Solu-Medrol®, Sorafenib, SPRYCEL™, STI-571, Streptozocin, SU11248, Sunitinib, Sutent®, Tamoxifen, Tarceva®, Targretin®, Tasigna®, Taxol®, Taxotere®, Temodar®, Temozolomide, Temsirolimus, Teniposide, TESPA, Thalidomide, Thalomid®, TheraCys®, Thioguanine, Thioguanine Tabloid®, Thiophosphoamide, Thioplex®, Thiotepa, TICE®, Toposar®, Topotecan, Toremifene, Torisel®, Treanda®, Tretinoin, Trexall™, Trisenox®, TSPA, TYKERB®, VCR, Vectibix™, Velban®, Velcade®, VePesid®, Vesanoid®, Viadur™, Vidaza®, Vinblastine, Vinblastine Sulfate, Vincasar Pfs®, Vincristine, Vinorelbine, Vinorelbine tartrate, VLB, VM-26, Vorinostat, Votrient, VP-16, Vumon®, Xeloda®, Zanosar®, Zevalin™, Zinecard®, Zoladex®, Zoledronic acid, Zolinza, and Zometa®.

The small molecule drug can be an anti-inflammatory agent. Anti-inflammatory agents include corticosteroids (e.g., prednisone, cortisone, methylprednisolone) and non-steroidal anti-inflammatory drugs (NSAIDs) (e.g., aspirin, celecoxib, diclofenc sodium, flurbiprofen, fenoprofen calcium, ibuprofen, indomethacin, ketoprofen, naproxen, oxaprozin, piroxicam, rofecoxib, sulindac, tolmetin sodium, and valdecoxib).

The small molecule drug can be an anti-infective agent. Anti-infective agents include antibacterial antibiotics, antivirals, and antifungals, and paraciticides.

A sugar may be added to the pulmonary air leakage occluding agent of the invention to improve the osmotic pressure of the solution from hypotonicity to isotonicity without reducing the pulmonary air leakage occluding effect, thereby allowing the biological safety to be increased.

The pulmonary air leakage occluding agent of the invention may be in the form of a powder, a solution, a gel, or the like. Since the self-assembling peptide gelates in response to changes in solution pH and salt concentration, it can be distributed as a liquid drug that gelates upon contact, or shortly following contact, with the body during application.

Formulations for clinical use can include cylinder-equipped syringes or pipettes that are prefilled with chemical solution containing components such as self-assembling peptides (prefilled syringes), or methods of supplying a chemical solution to a syringe or pipette chip by means that supplies the components through the opening of the syringe or pipette chip (an aspirator or valve), and applying it to the affected area through the discharge section. A construction with two or more syringes or pipettes is sometimes used.

The components may be used as a coating on an instrument such as a stent or catheter, to suppress pulmonary air leakage.

Also, the components may be anchored on a support such as gauze or a bandage, or a lining, that is commonly used in the field. The components may also be soaked into a sponge for use.

In addition, an atomizing sprayer filled with a powder or solution of the components may be prepared. When such a spray is used for spraying onto an affected area, the pH and salt concentration increase upon contact with the body, thereby causing gelling, and therefore this form can be applied for a great variety of sites and conditions.

An aspect of the invention concerns a method of treating a pulmonary air leak. The method includes the step of applying to a site of pulmonary air leak an effective amount of a peptide in accordance with the invention, i.e., an amphiphilic peptide having 8-200 amino acid residues with the hydrophilic amino acids and hydrophobic amino acids alternately bonded, and is a self-assembling peptide exhibiting a beta-sheet structure in aqueous solution in the presence of physiological pH and/or in the presence of a cation.

As used herein, a "pulmonary air leak" refers to any situation in which air abnormally escapes from airways of the lung, for example, into the extra-alveolar spaces. Pulmonary air leaks can occur spontaneously in conditions such as emphysema, in which blebs rupture. Pulmonary air leaks also can occur as a result of trauma (penetrating or non-penetrating) to the chest, as well as surgical procedures (and complications thereof) involving the lungs. In one instance, a pulmonary air leak may present as pulmonary interstitial emphysema, pneumomediastinum, pneumothorax, pneumopericardium, pneumoperitoneum, subcutaneous emphysema, or any combination thereof. In one instance, a pulmonary air leak may occur in association with surgical biopsy or resection of lung tissue, for example, resection of small cell lung cancer, carcinoid tumor, non-small cell lung cancer, adenocarcinoma, or squamous cell carcinoma.

As used herein, "applying" is locally administering, for example by soaking, dripping, painting, spraying, or otherwise contacting a tissue site to be treated. In one instance, the site is lung parenchymal tissue, e.g., at a site of resection. In one instance, the site is a trachea, bronchus, bronchiole, or other airway. In one instance, the site is a bronchus.

In one embodiment the peptide is applied thoracoscopically, i.e., via a thoracoscopic instrument. Such instruments are well known in the art and need not be described further here. In one instance, the peptide is applied during thoracoscopic surgery.

In one embodiment the peptide is applied bronchoscopically, i.e., via a bronchoscopic instrument. Such instruments are well known in the art and need not be described further here. In one instance, the peptide is applied during bronchoscopic surgery or during a bronchoscopic procedure such as a bronchoscopic examination, bronchoscopic biopsy, bronchoscopic brushing, or bronchoscopic alveolar lavage.

The peptide is applied in an effective amount to treat the pulmonary air leak. As used herein, the term "treat" means to reduce, ameliorate, or cure a condition of a subject. A "subject" as used herein refers to a mammal, specifically including but not limited to a human.

An "effective amount" as used herein is an amount that is sufficient to bring about a desired biological result. Persons skilled in the art will have no difficulty ascertaining what constitutes an effective amount, based on conventional animal studies (such as described below) and/or clinical experience. An effective amount may vary depending on the particular lesion to be treated. For example, an effective amount may vary depending on factors such as the site of the lesion, the size of the lesion, the condition of the subject, and other factors readily recognized by ordinarily skilled practitioners.

In one instance, the peptide can be provided as an aqueous solution, in one instance the aqueous solution of peptide is about 0.5 % to about 3 % (w/v). The solvent for the aqueous solution can be water alone, physiologically isotonic dextrose (e.g. 5 % dextrose in water), physiologic saline, Ringer's solution, or the like. Other physiologically acceptable aqueous solvents are also embraced by the invention.

An aspect of the disclosure comprising the invention is a pharmaceutical composition comprising a peptide of the invention and a pharmaceutically acceptable carrier. A pharmaceutical composition can be made by combining a peptide of the invention and a pharmaceutically acceptable carrier. In one instance, the pharmaceutical composition is sterilized by any suitable method, e.g., sterile filtering. In one instance, the pharmaceutically acceptable carrier is selected from water alone and physiologically isotonic dextrose (e.g. 5% dextrose in water). In one instance, the pharmaceutical composition further includes at least one additional agent, for example a preservative, a stabilizing agent, or a coloring agent.

An aspect of the disclosure is a kit. The kit includes a peptide of the invention, an applicator, and instructions for use of the peptide and the applicator to occlude a pulmonary air leak. In one instance, the peptide of the invention is provided as a powder. In one instance, the peptide of the invention is provided as a powder and the kit further includes an aqueous solvent for the peptide. In one instance, the peptide is provided as an aqueous solution. In one instance, the applicator is a sponge. In one instance, the applicator is a dropper, for example with a deformable bulb and a tip through which a solution of the peptide can be drawn up and dispensed. In one instance, the applicator is constructed and arranged to dispense a solution of the peptide as a spray.

The pulmonary air leakage occluding agent of the invention will now be explained in greater detail through the following example.

### EXAMPLE

### Effects of 2.5 % Aqueous Peptide Solution in Miniature Swine Model

A miniature swine model of pulmonary air leak was used in an experiment to determine if a 2.5 % (w/v) aqueous solution of peptide could occlude the air leak. A pulmonary air leak was surgically created in at least one miniature swine ("mini-pig"). A 2.5 % aqueous solution of a self-assembling peptide in accordance with the invention was topically applied to the site of the air leak. Evaluation of the air leak showed it was occluded following application of the peptide solution.

The body weight of miniature swine (Göttingen) at receipt ranged from 21.4 to 22.6 kg. Animals were quarantined for 7 days and acclimatized for 2 days. The animal room was maintained at temperature 23 °C, 55 % humidity, lighting period of 12 hours (6:00 to 18:00), and ventilation 10 complete exchanges/hour (fresh air through filter).

**Study Design**

| | Number of animals | Animal number |
|---|---|---|
| Observation Group | 3 | M00001, M00002, M00003 |
| Necropsy in surgery | 1 | - |

### Diet

Animals were supplied with 500 g ± 5 g/day of pellet diet (manufactured within 5 months, Nisseiken, Ltd.) by using metal feeder in morning.

### Drinking water

Animal has free access to tap water using an automatic watering system.

### Anesthesia and treatment of pre-operation

Animals were anesthetized by intramuscular injection of 0.05 mg/kg atropine sulfate and 15 mg/kg ketamine hydrochloride in cervical back. A tracheal cannula (PORTEX) was inserted under general anesthesia provided as N₂O:O₂=1:1 mixture gas + 0.5 % isoflurane using inhalation apparatus (Vigor21 II, ACOMA Medical Industry Co., Ltd). Artificial respiration was carried out as follows: 10-15 mL/kg, 18-22 breaths/minute using artificial respirator (PRO-V mkII, ACOMA Medical Industry Co., Ltd). Further, Ampicillin + glucose lactated Ringer's solution (1 drop/second) was administered intravenously from pre-operation until closing of the chest.

### Thoracotomy and surgical creation of lung injury

Animals treated as above were positioned in left side lying position and lung was exposed by thoracotomy from the right side. An air leak was created by the perpendicular puncture in pulmonary lobe using 18G injection needle (TERUMO Ltd.). Confirmation of pulmonary air leakage was carried out with physiological saline solution that filled thoracic cavity. Then 10-15 mL of 2.5 % (w/v) self-assembling peptide hydrogel RADA16 was applied several times until the air leakage was stopped. Due to dispersion of the peptide by inflation and deflation of the lung, the self-assembling peptide was applied to the lesion with the assistance of the grip of tweezers to help localize the gel on the surface of the lung at the site of the puncture lesion. This procedure was effective to occlude the pulmonary air leakage.

The occlusion of pulmonary air leakage was initially checked by raising the internal pressure of the artificial respiration system. Then, the occlusion was finally confirmed by gradual increase of air pressure to 20 cm H₂O of internal pressure.

A lung sample was obtained from an animal which underwent necropsy in the surgery and fixed with 10 vol % neutralize buffered formalin to confirm the pulmonary air leak occlusion by self-assembling peptide hydrogel during the surgery. In the other three animals, the chest was sewed up with a chest tube temporarily left in place until extraplural air was fully evacuated.

A catheter (12G cannula, LCV-UK kit, Nippon Sherwood Ltd) was inserted into cranial sinus of venae cava for post-operative monitoring. Animals were recovered from anesthesia after the cannula was installed.

### Histopathological examination

The lung sample from the animal which underwent necropsy in surgery was fixed with formalin, sectioned, stained with hematoxylin and eosin, and examined by light microscopy in order to evaluate the site that had been punctured and then sealed with peptide.

### Postoperative care

500 mL of Viccillin+LactecD was administered twice daily (morning and afternoon) for 3 days from 1 day after operation. Ampicillin was administered by intramuscular injection in cervical back on day 4 after operation. Buprenophine (0.01 mg/kg) as a painkiller was injected intramuscularly into cervical back for 4 days following the operation.

### Evaluation

Chest X-ray examinations were conducted in pre-operation, post-operation and pre-necropsy using surgical x-ray TV equipment (DHF-105CX-PC, Hitachi Medico Ltd.).

All animals were observed for general appearance and death once a day from the day of experiment to the day of necropsy.

All animals were weighed with a digital platform scale (DUE600ST/ID3s-A, Mettler Toledo Ltd.) on the day of operation, 5 days post-operation, and the day of autopsy.

Food consumption was checked every day. Any remaining amount of food was weighed with a digital platform scale (DUE600ST/ID3s-A, Mettler Toledo Ltd.) when present. If there was no uneaten diet, food consumption was recorded as 500 g.

Blood for hematological examination was collected by catheter on the day of operation, 3 days after operation, and 7 days after operation, and the day of necropsy. Blood was collected into EDTA-2K coated blood collection tubes (VP-DK052K05, TERUMO Ltd.). Red blood cell count (RBC), white blood cell count (WBC), hemoglobin concentration (HGB), hematocrit (HCT), and platelet count (PLT) were measured using a multi-channel blood cell counter (Sysmex K-4500, Sysmex Ltd.).

Blood for biochemical examination was centrifuged at 3,000 rpm at 4 °C for 15 min to obtain serum samples used to measure AST, ALT, ALP, total protein (TP), albumin (Alb), protein fraction (alb), alpha 1 globulin (α₁-glb), alpha 2 globulin (α₂-glb), beta globulin (β-glb), gamma globulin (y-glb), albumin/globulin ratio (A/G), total bilirubin (T-Bil), urea nitrogen (UN), creatinine (CRE), glucose (Glu), total cholesterol (T-Cho), triglycerides (TG), sodium, potassium, chloride, calcium, inorganic phosphate (IP), and C-reactive protein (CRP).

### Necropsy

Animals were anesthetized by injection of 6.4 % pentobarbital sodium into auricular veins. Then, animals were euthanized by exsanguination by cutting the carotid artery.

### Results

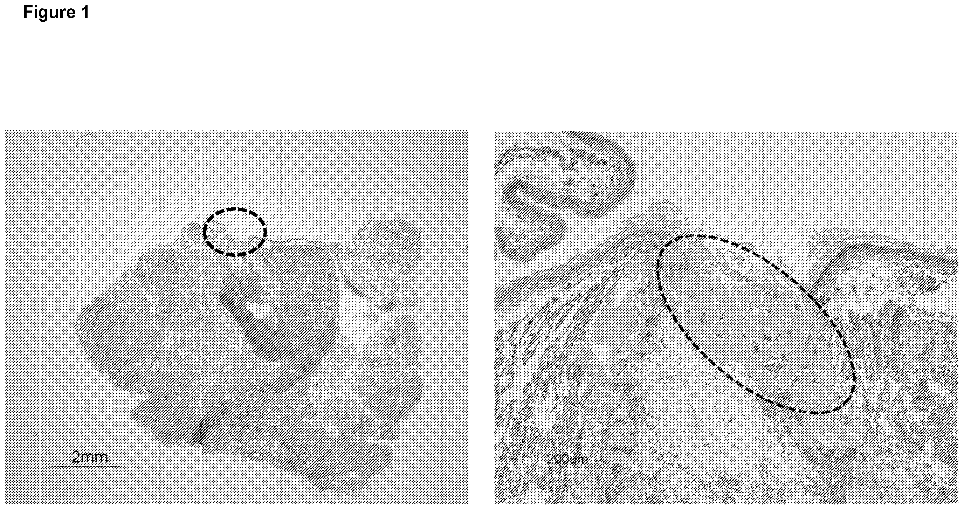

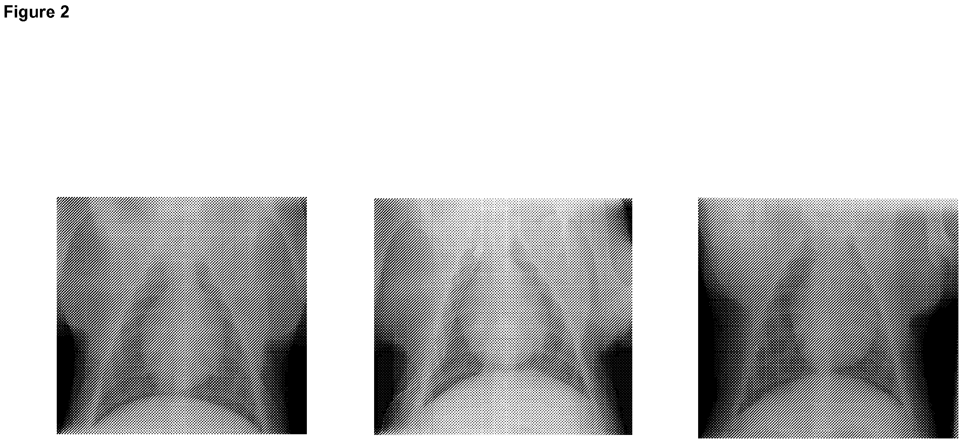

Pulmonary air leak was not found in any of the animals during and following treatment with peptide. Representative results of X-ray examination are shown in **Fig. 1****.** As shown in Fig. 1, no abnormality was found in lung from the day of operation to the day of necropsy. Representative results of histopathological examination are shown in **Fig. 2****.** As shown in Fig. 2, occlusion of pulmonary air leak by self-assembling peptide hydrogel was identified in histopathological examination.

All animals remained in good general condition throughout the experiment. Animals generally maintained body weight and food intake.

Results of hematological examination are shown in **Table 1.** All animals showed elevated level of WBC on day 3 after operation, and M0001 showed the high WBC on the day of necropsy. However, these changes were considered to be due to the open surgery but not due to the self-assembling peptide hydrogel.

Results of biochemical examination are shown in **Table 2.** An elevated level of CRP was evident on day 3 after operation. The changes in CRP and in other parameters were considered to be due to the open surgery but not due to the self-assembling peptide hydrogel.

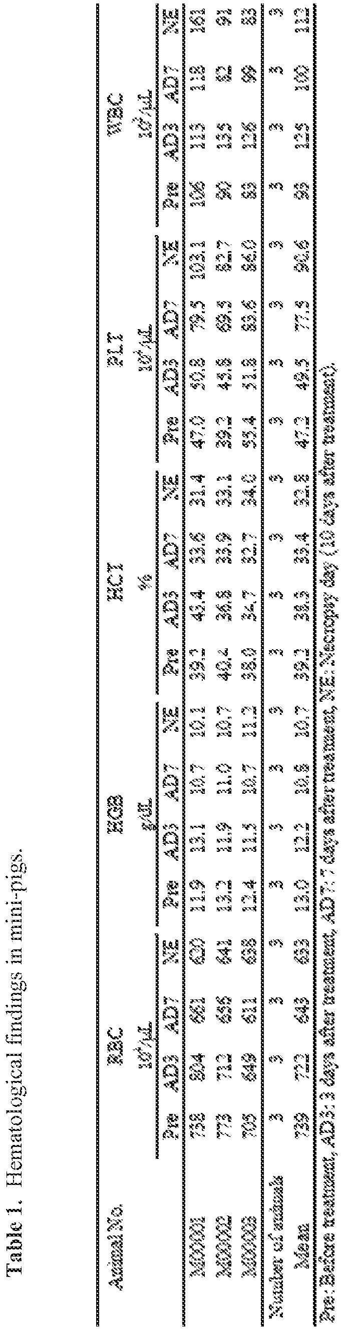

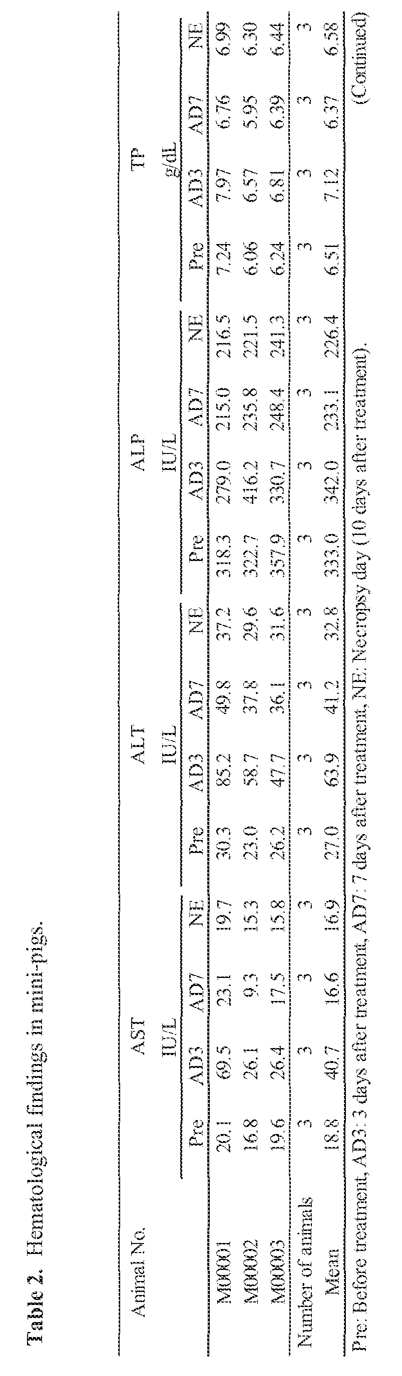

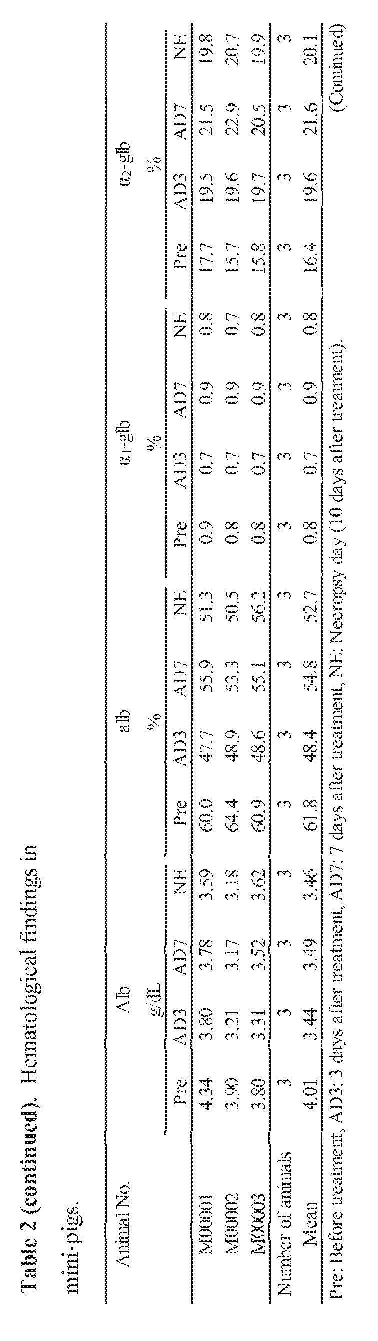

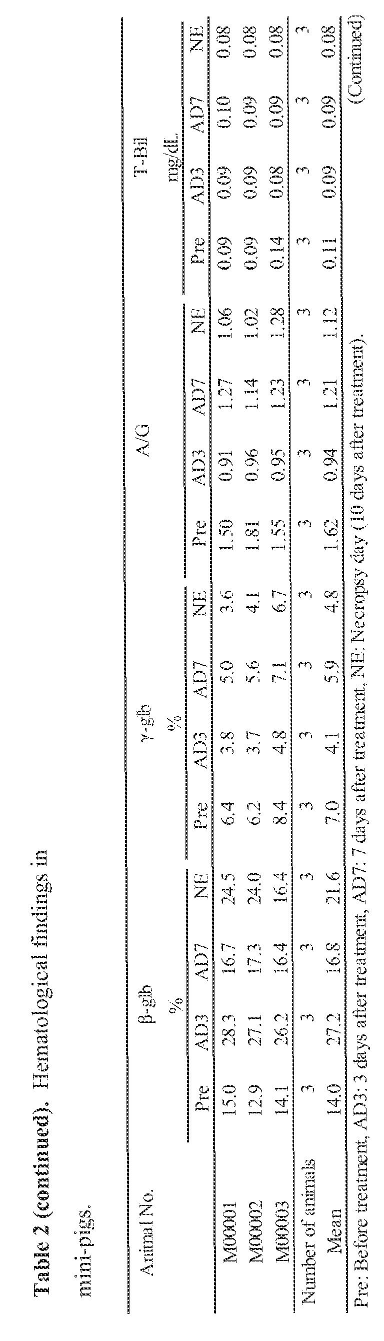

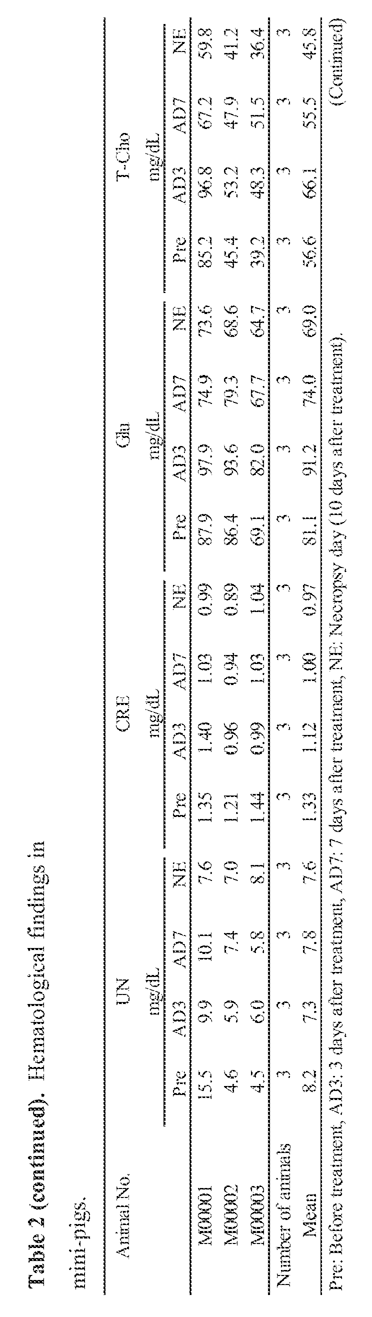

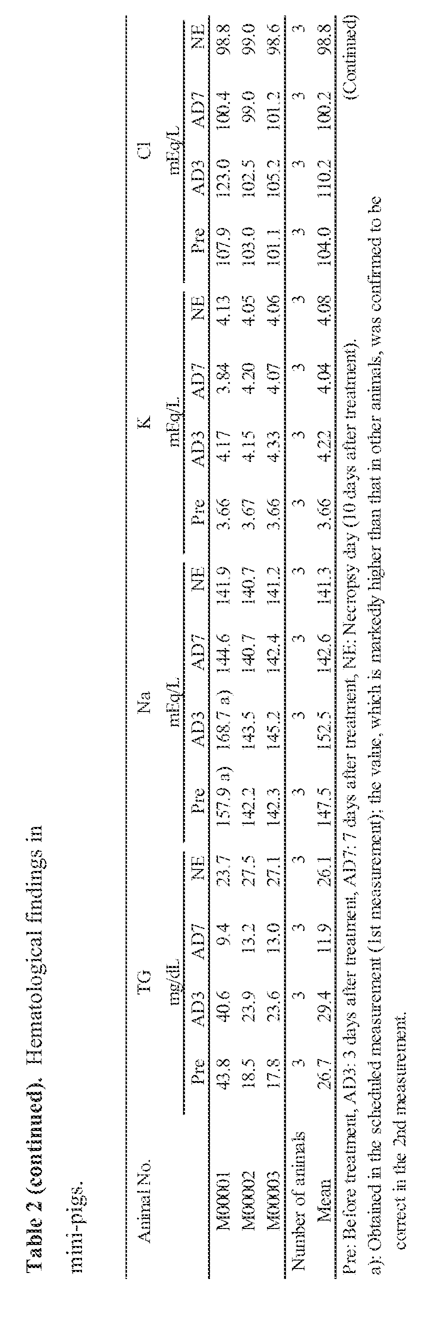

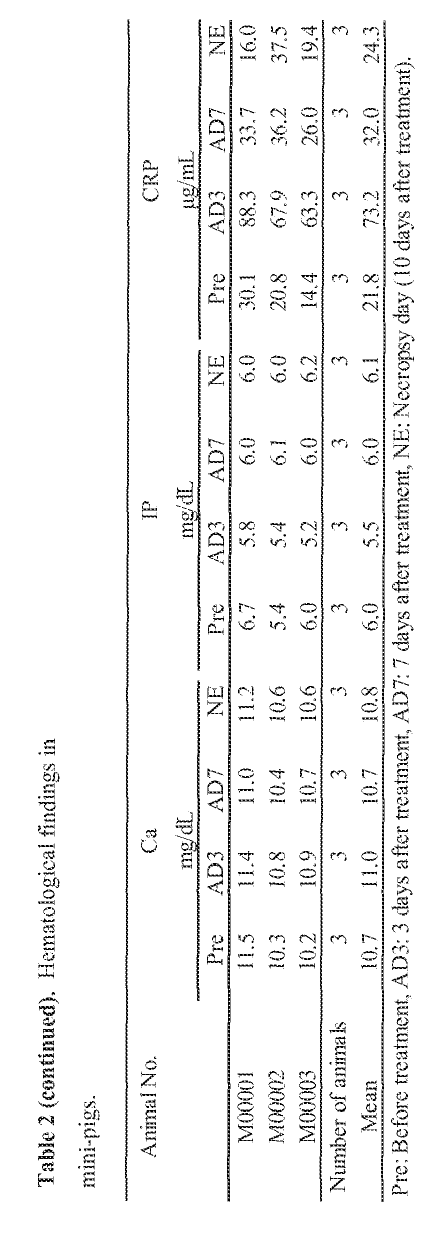

## Claims

1. A peptide for use in occluding pulmonary air leakage, wherein the peptide is an amphiphilic peptide of the amino acid sequence Ac-(RADA)4-CONH2 (SEQ ID NO:1), comprising amino acid residues with the hydrophilic amino acids and hydrophobic amino acids alternately bonded, wherein the peptide is a self-assembling peptide exhibiting a beta-sheet structure in aqueous solution in the presence of physiological pH and/or in the presence of a cation, and wherein the peptide is provided as an aqueous solution of 2.5 % (weight of peptide to volume).

2. The peptide for use of claim 1, wherein the peptide is applied to lungs.

3. The peptide for use of claim 1 or claim 2, wherein the peptide is for application to a bronchus.

4. The peptide for use of any one of claims 1-3, wherein the peptide is for application thoracoscopically.

5. The peptide for use of any one of claims 1-4, wherein the peptide is for application bronchoscopically.

6. The peptide for use of any one of claims 1-5, wherein the peptide is for administration in an agent, wherein the agent also includes at least one small molecule drug useful to treat a condition selected from the group consisting of cancer, inflammation, and infection, wherein the small molecule drug is selected from the group consisting of anti-cancer agents, corticosteroids, non-steroidal anti-inflammatory drugs, antibacterial antibiotics, antivirals, antifungals, and parasiticides.

## Patentansprüche

1. Peptid zur Verwendung beim Verschließen einer Lungenluftleckage, wobei das Peptid ein amphiphiles Peptid der Aminosäuresequenz Ac-(RADA)4-CONH2 (SEQ ID NO:1) ist, umfassend Aminosäurereste mit abwechselnd gebundenen hydrophilen Aminosäuren und hydrophoben Aminosäuren, wobei das Peptid ein selbstanordnendes Peptid ist, das in wässriger Lösung in der Anwesenheit eines physiologischen pH-Werts und/oder in der Anwesenheit eines Kations eine beta-Faltblatt-Struktur zeigt, und wobei das Peptid als eine wässrige Lösung von 2,5 % (Gewicht des Peptids zum Volumen) bereitgestellt wird.

2. Peptid zur Verwendung nach Anspruch 1, wobei das Peptid auf die Lunge angewendet wird.

3. Peptid zur Verwendung Anspruch 1 oder Anspruch 2, wobei das Peptid zur Anwendung auf einen Bronchus dient.

4. Peptid zur Verwendung nach einem der Ansprüche 1-3, wobei das Peptid zur thorakoskopischen Anwendung dient.

5. Peptid zur Verwendung nach einem der Ansprüche 1-4, wobei das Peptid zur bronchoskopischen Anwendung dient.

6. Peptid zur Verwendung nach einem der Ansprüche 1-5, wobei das Peptid zur Verabreichung in einem Mittel dient, wobei das Mittel ebenfalls mindestens ein niedermolekulares Arzneimittel beinhaltet, das hilfreich bei der Behandlung eines Zustands ist, der ausgewählt ist aus der Gruppe bestehend aus Krebs, Entzündung und Infektion, wobei das niedermolekulare Arzneimittel ausgewählt ist aus der Gruppe bestehend aus Mitteln gegen Krebs, Corticosteroiden, nichtsteroidalen Antirheumatika, antibakteriellen Antibiotika, antiviralen Mitteln, Antimykotika und Parasitiziden.

## Revendications

1. Peptide pour utilisation dans l'occlusion de fuite d'air pulmonaire, ledit peptide étant un peptide amphiphile de la séquence d'acides aminés Ac-(RADA)4-CONH2 (SEQ ID n° : 1), comprenant des résidus d'acides aminés, les acides aminés hydrophiles et les acides aminés hydrophobes étant liés de façon alternée, ledit peptide étant un peptide à auto-assemblage présentant une structure en feuillets bêta en solution aqueuse en présence d'un pH physiologique et/ou en présence d'un cation, et ledit peptide étant fourni sous la forme d'une solution aqueuse de 2,5 % (poids de peptide par volume).

2. Peptide pour utilisation selon la revendication 1, ledit peptide étant appliqué sur les poumons.

3. Peptide pour utilisation selon la revendication 1 ou 2, ledit peptide étant destiné à une application sur une bronche.

4. Peptide pour utilisation selon l'une quelconque des revendications 1 à 3, ledit peptide étant destiné à une application par thoracoscopie.

5. Peptide pour utilisation selon l'une quelconque des revendications 1 à 4, ledit peptide étant destiné à une application par bronchoscopie.

6. Peptide pour utilisation selon l'une quelconque des revendications 1 à 5, ledit peptide étant destiné à une administration dans un agent, ledit agent comprenant également au moins un médicament à petites molécules utile pour traiter un état choisi dans le groupe constitué par un cancer, une inflammation et une infection, ledit médicament à petites molécules étant choisi dans le groupe constitué par des agents anticancéreux, des corticoïdes, des médicaments antiinflammatoires non stéroïdiens, des antibiotiques antibactériens, des antiviraux, des antifongiques et des parasiticides.
